# EUROPÄISCHE PATENTANMELDUNG

(11) **EP 2 524 716 A1**
(43) Veröffentlichungstag der Anmeldung: **21.11.2012**
(21) Anmeldenummer: 11005148.9
(22) Anmeldetag: 24.06.2011
(51) Int. Cl.: A61N 1/05, A61M 19/00, A61H 39/06

(54) **Stimulationsvorrichtung**

(30) Priorität: 14.05.2011 DE 102011101662
(71) Anmelder: cerboMed GmbH, 91052 Erlangen (DE)
(72) Erfinder: Hartlep, Andreas, 83607 Holzkirchen (DE); Ellrich, Jens, 91094 Langensendelbach (DE)
(74) Vertreter: Gosdin, Michael

(57) **Zusammenfassung**

Die Erfindung betrifft eine Stimulationsvorrichtung (1) zur Stimulation mindestens eines rezeptiven Feldes eines Hautnervs des Kopf- oder Halsbereichs eines Menschen, wobei die Stimulationsvorrichtung (1) mindestens ein Stimulationselement (2) umfasst, das mit am Kopf (K) des Menschen anbringbaren Haltemitteln (3) in einer Lage gehalten werden kann, so dass eine Stimulationsoberfläche (4) des Stimulationselements (2) auf mindestens einem rezeptiven Feld aufliegt. Hiermit wird es möglich, eine effiziente Nervenstimulation zu erreichen.

## Beschreibung

Die Erfindung betrifft eine Stimulationsvorrichtung zur Stimulation mindestens eines rezeptiven Feldes eines Hautnervs des Kopf- oder Halsbereichs eines Menschen.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zu invasiven als auch zu non-invasiven Stimulation.

Bekannt ist in verschiedenen Variationen insbesondere die Stimulation des Vagusnervs mittels einer Stimulationsvorrichtung, die eine transkutane elektrische Stimulation vornimmt.

Die DE 10 2005 003 735 B4 offenbart eine Stimulationsvorrichtung, die einen bügelförmigen Fortsatz aufweist, an dessen Ende ein Elektrodenkopf samt Elektroden angeordnet ist. Der bügelförmige Fortsatz wird in den Gehörgang eingeführt, über die Wandung des Gehörkanals wird dann der Vagusnerv stimuliert.

In der DE 10 2006 023 824 B4 wird vorgesehen, die Stimulationsvorrichtung in der Pinna anzuordnen. Dabei können Elektroden eingesetzt werden, die am Ende elastischer Spannelemente positioniert sind, mit denen die Vorrichtung in der Pinna gehalten wird.

Es hat sich herausgestellt, dass die konventionelle transkutane Stimulation der sensiblen Hautnerven an Gesicht, Kopf und Hals bei einer Vielzahl von neurologischen, psychiatrischen, internistischen und Hals-Nasen-Ohren-Erkrankungen nicht ausreicht. Demgemäß sind die vorbekannten Stimulationssysteme gegebenenfalls für die genannte Anwendung, aber auch für andere Anwendungen, nicht optimal.

Der Erfindung liegt daher die **Aufgabe** zugrunde, eine Stimulationsvorrichtung zur Stimulation der rezeptiven Felder der oben genannten sensiblen Nerven des Kopf- bzw. Halsbereichs vorzusehen, mit der es möglich ist, bei Bedarf eine stärkere und effizientere Nervenstimulation zu erreichen.

Die **Lösung** dieser Aufgabe durch die Erfindung ist dadurch gekennzeichnet, dass die Stimulationsvorrichtung zur Stimulation mindestens eines rezeptiven Feldes eines Hautnervs des Kopf- oder Halsbereichs eines Menschen ausgebildet ist, indem die Stimulationsvorrichtung mindestens ein Stimulationselement umfasst, das mit am Kopf des Menschen anbringbaren Haltemitteln in einer Lage gehalten werden kann, so dass eine Stimulationsoberfläche des Stimulationselements auf mindestens einem rezeptiven Feld aufliegt.

Die Stimulationsoberfläche des Stimulationselements liegt dabei bevorzugt flächig, insbesondere mit einer Auflagefläche zwischen 0,1 cm² und 200 cm², besonders bevorzugt mit einer Auflagefläche zwischen 2 cm² bis 150 cm², auf einem definierten Hautbereich auf.

Das Stimulationselement kann mindestens eine elektrische Elektrode zur Aufbringung eines transkutanen elektrischen Stimulationsreizes auf den definierten Hautbereich aufweisen.

Eine alternative - gegebenenfalls aber auch additive - Ausgestaltung sieht vor, dass das Stimulationselement mindestens ein wärmeerzeugendes und/oder kälteerzeugendes Element zur Einleitung von Wärme und/oder Kälte auf den definierten Hautbereich aufweist.

Eine weitere alternative - und wiederum gegebenenfalls auch additive - Konzeption sieht vor, dass das Stimulationselement mindestens ein Schwingungen erzeugendes Element zur Einleitung von Vibrationen auf den definierten Hautbereich aufweist.

Die Stimulationsoberfläche des Stimulationselementes kann zumindest auf einem Teil des Hautareals über dem Processus mastoideus aufliegen.

Gemäß einer möglichen Ausgestaltung umfassen die Haltemittel einen am Stimulationselement angeordneten oder angeformten bügelförmigen Abschnitt, der ausgebildet ist, um hinter der Pinna des Ohrs eingehängt zu werden.

Die Haltemittel können weiterhin einen am Stimulationselement angeordneten oder angeformten Haltebügel umfassen, der ausgebildet ist, um einen Teil des Kopfs oder Hals, insbesondere um den Hinterkopf, umzulaufen und mindestens ein Stimulationselement, vorzugsweise zwei Stimulationselemente für zwei rezeptive Felder auf jeder Seite des Kopfs, in Position zu halten.

Der Haltebügel kann dabei so ausgebildet sein, dass er bei bestimmungsgemäßem Gebrauch der Stimulationsvorrichtung das mindestens eine Stimulationselement, vorzugsweise zwei Stimulationselemente, mit Vorspannung an die Oberfläche des Kopfs drückt.

Die Haltemittel umfassen gemäß einer weiteren Ausführungsform der Erfindung einen schalenförmigen Körper, der eine konkave Oberfläche aufweist, um einen Abschnitt des Kopfes zu umfassen. Der schalenförmige Körper kann dabei aus einem Material bestehen (z. B. aus Metall), das plastisch verformbar ist. Damit kann der schalenförmige Körper der individuellen Kopfform angepasst werden. In der plastisch umgeformten Lage kann das Material dann aber auch wieder elastische Eigenschaften aufweisen, um eine elastische Anlagekraft auf die Oberfläche des Kopfes an den gewünschten Behandlungsstellen zu erzeugen.

Die Haltemittel können auch einen bandförmigen Streifen umfassen, an dem mindestens ein Stimulationselement angeordnet ist, wobei der bandförmige Streifen insbesondere ausgebildet ist, über einen Teil des Kopfs flächig zu verlaufen, wobei der bandförmige Streifen insbesondere aus einem Material besteht, das plastisch verformbar ist. Der bandförmige Streifen kann mindestens eine Gabelung aufweisen, so dass er mindestens drei Abschnitte hat, wobei insbesondere jeder Abschnitt bei bestimmungsgemäßem Gebrauch des Stimulationselements über einen Umfangsabschnitt des Kopfs verläuft.

Die Haltemittel sind insbesondere so ausgebildet, dass mindestens ein Stimulationselement in mindestens einem der folgenden Bereiche des Kopfs gehalten bzw. positioniert werden kann:
- Bereich des rezeptiven Feldes des Nervus ophthalmicus (erster Ast des Nervus trigeminus) im Bereich der Stirn und des Scheitels,
- Bereich des rezeptiven Feldes des Nervus maxilliaris (zweiter Ast des Nervus trigeminus) im Bereich der Wange und oberen Schläfe,
- Bereich des rezeptiven Feldes des Nervus mandibularis (dritter Ast des Nervus trigeminus) am Kinn, Unterkiefer und unterer Schläfe bis oberhalb des Ohres,
- Bereich des rezeptiven Feldes des Nervus auricularis magnus vom seitlichen Unterkiefer bis hinter das Ohr,
- Bereich des rezeptiven Feldes des Nervus occipitalis minor hinter dem Ohr bis zur Hinterhauptschuppe,
- Bereich des rezeptiven Feldes des Nervus occipitalis major an der Hinterhauptschuppe,
- Bereich des rezeptiven Feldes der Hautäste des Nervus cervicalis III im Halsbereich von unterhalb der Kinnspitze bis zu den Sehnenansatzpunkten an der Hinterhauptschuppe,
- Bereich des rezeptiven Feldes des Nervus vagus, insbesondere des aurikulären Vagusnervs, im Bereich der Pinna und über dem Mastoid.

Die Haltemittel können dabei insbesondere so ausgebildet sein, dass mindestens ein Stimulationselement jeweils auf dem linken und rechten rezeptiven Feld gehalten werden kann.

Bei Bedarf kann die vorgeschlagene Stimulationsvorrichtung auch mit einer weiteren Stimulationsmöglichkeit kombiniert werden, wonach sie weiterhin ein Stimulationselement umfasst, das am oder im Ohr anbringbar ist und das zur vorzugsweise transkutanen elektrischen Stimulation eines weiteren Abschnitts des Ohrs, insbesondere im Bereich der Cymba conchae, ausgebildet ist.

Da der genaue anatomische Verlauf der Hautäste im jeweiligen rezeptiven Feld einer großen Variabilität unterliegt, ist darauf zu achten, gegebenenfalls dessen gesamten Bereich mit dem Stimulationselement abzudecken. Es wird hierzu also eine möglichst große Fläche bis hin zu den anatomischen Grenzen des jeweiligen Versorgungsgebietes für die Stimulation vorgesehen.

Für die optimale Stimulation werden geeignete Stromformen und Stimulationssequenzen vorgesehen, mit denen die in der Haut endigenden Nervenbündel beeinflusst werden können.

Dabei haben sich Stimulationsfrequenzen im Bereich zwischen 1 und 100 Hz bewährt. Die Phasendauer von Stromimpulsen im Falle einer elektrischen Stimulation bewegt sich zumeist zwischen 10 und 1.000 µs.

Vorgesehen ist insbesondere eine noninvasive, d. h. transkutane elektrische Nervenstimulation. Primär geeignet ist hierfür das Stimulationselement, das sich flächig auf den zu stimulierenden Bereich auflegt. Erwähnt wurde hierzu bereits die Positionierung mittels eines elastischen Haltebügels. Als Haltemittel wären aber beispielsweise auch Klebeelektroden möglich, die gegebenenfalls mit mindestens einer räumlich entfernten, größeren Gegenelektrode kombiniert werden.

Vorgesehen werden kann mindestens eine Stimulationselektrode, wobei eine Referenz-, Gegen- bzw. Neutralelektrode auch an einer entfernteren Stelle den Körper kontaktieren kann.

Möglich ist die Stimulation auf nur der linken oder rechten Kopfseite, aber auch an beiden Seiten, jeweils an korrespondierenden Stellen.

Oben erwähnt wurde bereits ein beidohrig anzulegender Bügel (Haltebügel), der mit einem nach vorne gebogenen Haken (bügelförmiger Abschnitt) über bzw. hinter den Ohrmuscheln eingehängt und hinter dem Nacken vom einen zum anderen Ohr geführt wird.

Der jeweils für die Stimulation vorgesehene, d. h. die Elektroden tragende Teil der Stimulationsvorrichtung ist zur Bildung des Stimulationselements flächig verbreitert und trägt die Elektroden für den Bereich des jeweiligen rezeptiven Feldes.

So kann beispielsweise eine vom Haltebügel ausgehende flächige Stimulationselektrode das rezeptive Feld des Nervus occipitalis major am Hinterhaupt abdecken.

Als Alternative kann der genannte Haltebügel auch nach Art eines Kopfhörers ausgeführt sein und bei bestimmungsgemäßem Gebrauch über den Scheitel des Benutzers verlaufen. In diesem Fall können sich die jeweiligen Enden des über den Scheitel verlaufenden Haltebügels über der Ohrwurzel in einen vorderen und einen hinteren Anteil gabeln. Der vordere Anteil dient dabei lediglich der Halterung, der hintere Anteil umfasst das Stimulationselement, im Falle der elektrischen Reizung des Nervs also die Elektroden.

Die Stimulationselemente können gemäß einer alternativen Lösung auch in einen hinter den Ohren weiter nach hinten und unten gerührten Brillenbügel integriert sein, wobei wiederum eine ein- oder beidseitige Stimulation möglich ist.

Eine weitere alternative Lösung stellt auf eine magnetische Halterung, insbesondere mittels eines Permanentmagneten, in bzw. an der Pinna ab. Hier kann eine anatomisch angeformte Magnetelektrode zum Einsatz kommen.

Eine hinter dem Ohr anzuordnende Elektrode bzw. das Stimulationselement kann dabei zumindest teilweise magnetisiert sein. In diesem Falle kann ein ferritisches Gegenstück in der Cymba conchae des Ohrs angeordnet werden.

Analog kann aber auch eine Elektrode bzw. ein Stimulationselement im Ohr zumindest teilweise magnetisiert sein, wobei dann ein ferritisches Gegenstück hinter dem Ohr angeordnet ist.

Die genannten Gegenstücke können jeweils selbst Elektroden tragen. Möglich ist es auch, dass - wie oben bereits angesprochen - neben dem Stimulationselement für die genannten Bereiche auch ein Stimulationselement mit zusätzlichen Elektroden an bzw. in der Pinna vorhanden ist, insbesondere in der Cymba conchae.

In der Zeichnung sind Ausführungsbeispiele der Erfindung dargestellt. Es zeigen:
- Fig. 1: den Kopf eines Menschen, an dem die hier interessierenden rezeptiven Felder der Nerven eingezeichnet sind, die simuliert werden sollen,
- Fig. 2: den Kopf in der Darstellung nach Fig. 1, wobei nun eine Stimulationsvorrichtung gemäß einer ersten Ausführungsform der Erfindung am Kopf angeordnet ist,
- Fig. 3: schematisch in perspektiver Ansicht den Kopf samt Ohr eines Menschen mit Markierung der für die Stimulation vorgesehenen Areale, um das rezeptive Feld des Vagusnervs zu stimulieren,
- Fig. 4: in der Darstellung nach Fig. 3 eine Stimulationsvorrichtung, die nunmehr am Kopf angeordnet ist, und
- Fig. 5: die Stimulationsvorrichtung nach Fig. 4, wobei die Ansicht in Richtung "X" dargestellt ist.

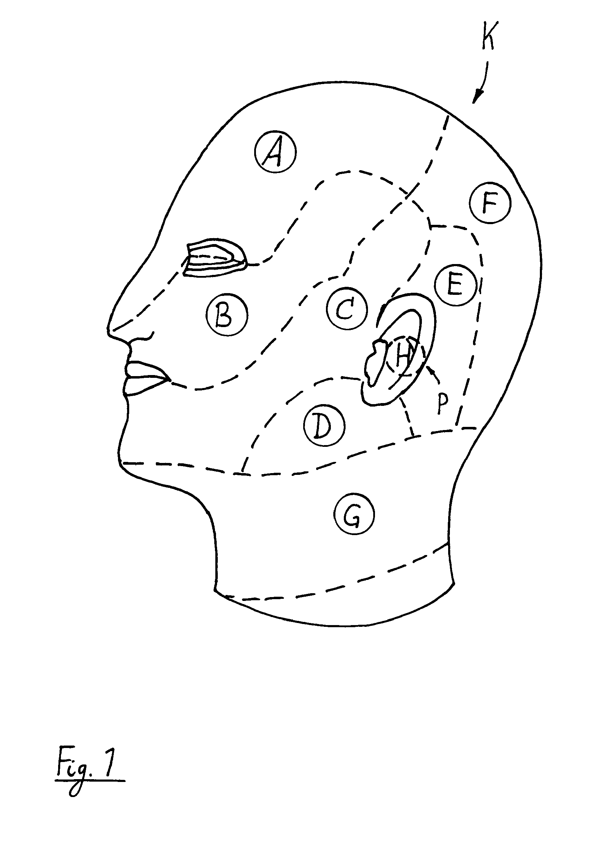

In Fig. 1 ist ein Kopf K eines Menschen zu sehen, an dem eine Stimulationsvorrichtung zur Stimulation mindestens eines rezeptiven Feldes eines Hautnervs im Kopf- und/oder Halsbereich angebracht werden soll.

In Frage kommen hierfür die folgenden in der Figur markierten rezeptiven Felder der folgenden Nerven:
- A: Nervus ophthalmicus (erster Ast des Nervus trigeminus)
- B: Nervus maxillaris (zweiter Ast des Nervus trigeminus)
- C: Nervus mandibularis (dritter Ast des Nervus trigeminus)
- D: Nervus auricularis magnus
- E: Nervus occipitalis minor
- F: Nervus occipitalis major
- G: Nervus cervicalis III
- H: Nervus vagus

Weiterhin ist das rezeptive Feld des Nervus vagus hinter der Pinna P des Ohrs für die Stimulation vorgesehen.

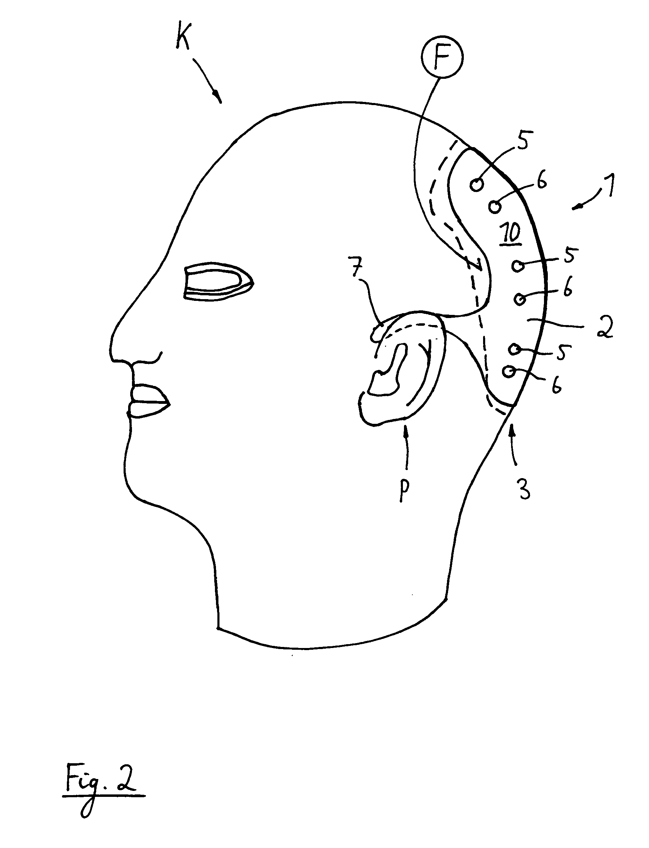

In Fig. 2 ist zu sehen, wie am Kopf K eine Stimulationsvorrichtung 1 angeordnet ist, um vorliegend das rezeptive Feld des Nervus occipitalis major F zu stimulieren.

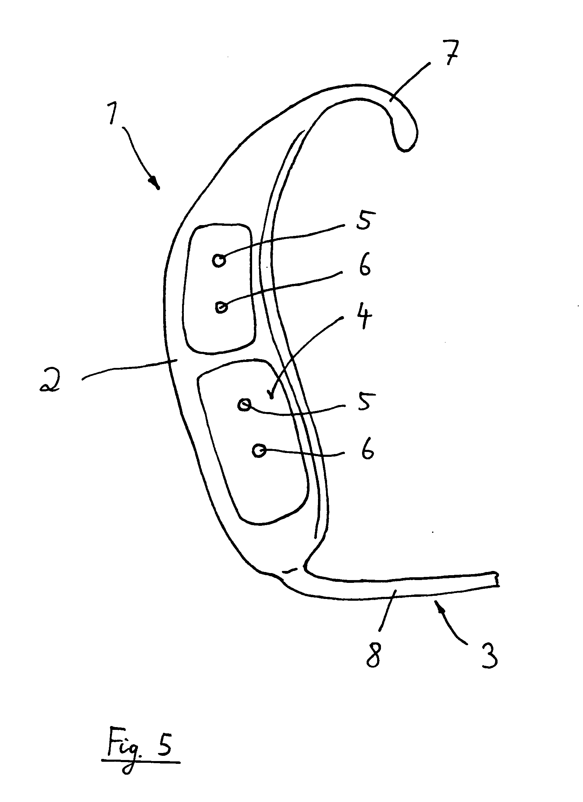

Die Stimulationsvorrichtung 1 weist hierzu ein flächig ausgebildetes Stimulationselement 2 auf, das eine solche flächige Ausdehnung aufweist, dass das zu stimulierende rezeptive Feld abgedeckt werden kann. Die Stimulationsoberfläche 4 ist exemplarisch in Fig. 5 illustriert. Jedes Stimulationselement 2 weist jeweils mindestens eine Stimulationselektrode 5 und mindestens eine zugeordnete Referenzelektrode 6 auf, zwischen denen in bekannter Weise ein elektrischer Strom fließt, um eine transkutane Nervenstimulation durchführen zu können.

Damit die Stimulationselemente 2 mit ihren Elektroden 5, 6 in der richtigen Position angeordnet und gehalten werden können, sind Haltemittel 3 vorgesehen, die im Ausführungsbeispiel gemäß Fig. 2 als schalenförmiger Körper 10 ausgeführt sind. Am schalenförmigen Körper 10 ist ein bügelförmiger Abschnitt 7 angeformt, mit dem der schalenförmige Körper 10 hinter die Pinna P beider Ohren eingehängt werden kann.

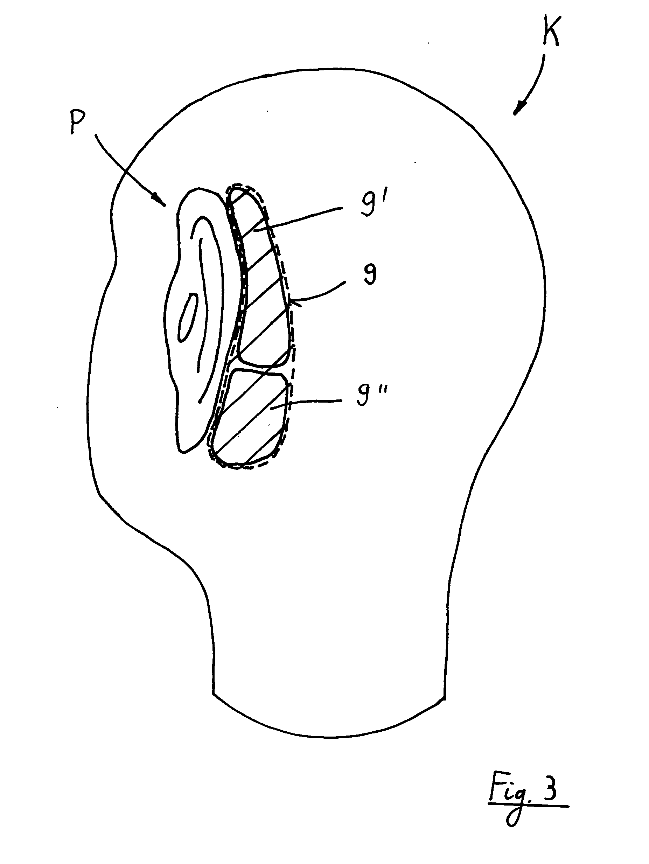

In Fig. 3 ist wiederum der Kopf K skizziert, wobei hier die Pinna P des Ohrs zu erkennen ist. Stimuliert werden soll hier gezielt der rezeptive Bereich des Vagusnervs. Dargestellt sind hier Bereiche hinter dem Ohr, die bestimmungsgemäß einer Stimulation unterzogen werden sollen.

Der insgesamt für die Stimulation in Frage kommende Bereich ist mit 9 markiert (s. gestrichelte Umrandung und den schraffierten Bereich). Der Bereich 9 umfasst allerdings zwei spezielle Bereiche, die spezifisch für die Stimulation vorgesehen werden können. Der eine Bereich 9' ist derjenige Abschnitt, wo der Stamm des Vagusnervs liegt. Der andere Bereich 9" liegt direkt über dem Felsenbein (Processus Mastoideus); demgemäß liegt hier eines der rezeptiven Felder zur Stimulation des Vagusnervs. Vorliegend wird der Bereich 9" zur Stimulation vorgesehen.

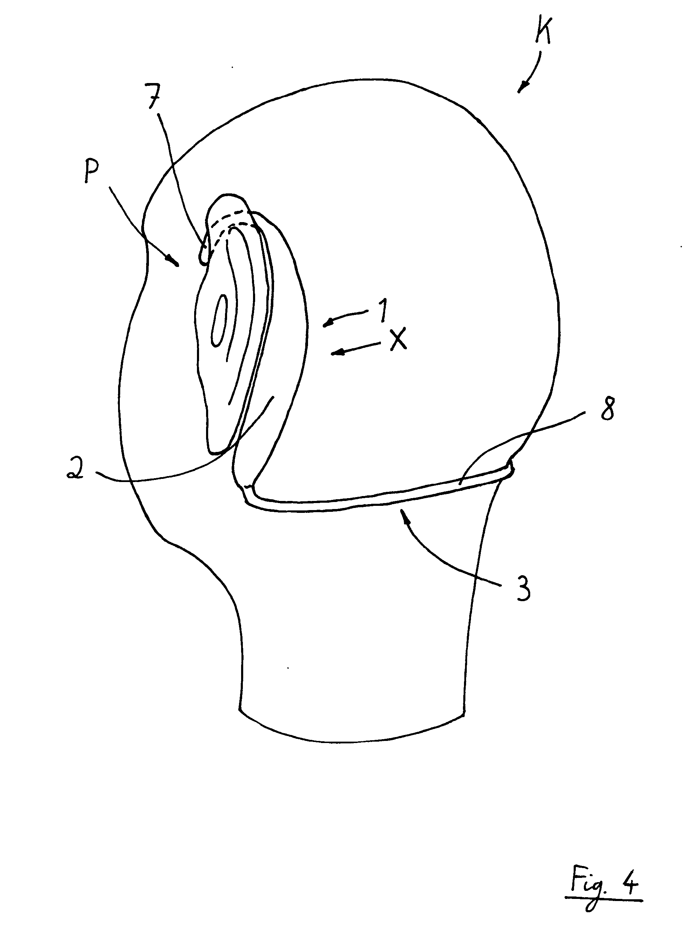

In Fig. 4 ist die Stimulationsvorrichtung 1 - jedenfalls ein Teil derselben - zu sehen, deren Kernstück wiederum ein Stimulationselement 2 ist. Das Stimulationselement 2 ist besonders gut in Fig. 5 zu erkennen, wo es - gesehen aus der Richtung X gemäß Fig. 4 - dargestellt ist. Das Stimulationselement 2 weist eine flächig ausgebildete Stimulationsoberfläche 4 auf, in der im Ausführungsbeispiel elektrische Elektroden 5 und 6 angeordnet sind. Die Darstellung der Elektroden 5, 6 in Fig. 5 ist nur sehr beispielhaft zu verstehen. Es kann eine Stimulation- und eine Referenzelektrode vorgesehen werden. Möglich ist es aber auch, dass mehrere Stimulations- und Referenzelektroden vorgesehen werden. Es ist auch möglich, dass eine oder mehrere Referenzelektroden an einem anderen Ort des Körpers platziert werden.

Das Stimulationselement 2 wird wiederum mit Haltemitteln 3 so in Position gehalten, dass bei bestimmungsgemäßem Gebrauch die Stimulationsoberfläche 4 des Stimulationselements 2 leicht an die Hautoberfläche des Bereichs 9, 9', 9" gedrückt wird. Im Ausführungsbeispiel weisen die Haltemittel 3 hierfür einen Haltebügel 8 auf, der um den Hinterkopf bzw. Nacken des Benutzers umläuft und zwei Stimulationselemente für beide Ohren unter leichter elastischer Vorspannung in Position hält.

Des weiteren ist an dem Stimulationselement 2 wieder ein bügelförmiger Abschnitt 7 angeordnet, der vorgesehen ist, um hinter die Pinna P gehängt zu werden. Beide Elemente 7 und 8 der Haltemittel 3 wirken dabei synergetisch und sorgen für eine bequeme und angenehme Positionierung der Stimulationsvorrichtung 1 auf dem vorgesehenen Bereich 9.

In das dargestellte Stimulationselement 2 kann die gesamte Elektronik zur Steuerung der Nervenstimulation samt Energieversorgung integriert sein.

### Bezugszeichenliste:

- 1: Stimulationsvorrichtung
- 2: Stimulationselement
- 3: Haltemittel
- 4: Stimulationsoberfläche
- 5: Elektrode
- 6: Elektrode
- 7: bügelförmiger Abschnitt
- 8: Haltebügel
- 9: Bereich (Stimulationsbereich)
- 9': Bereich (Stimulationsbereich)
- 9": Bereich (Stimulationsbereich)
- 10: schalenförmiger Körper

- K: Kopf
- P: Pinna

- A: rezeptives Feld des Nervus ophthalmicus (erster Ast des Nervus trigeminus)
- B: rezeptives Feld des Nervus maxillaris (zweiter Ast des Nervus trigeminus)
- C: rezeptives Feld des Nervus mandibularis (dritter Ast des Nervus trigeminus)
- D: rezeptives Feld des Nervus auricularis magnus
- E: rezeptives Feld des Nervus occipitalis minor
- F: rezeptives Feld des Nervus occipitalis major
- G: rezeptives Feld des Nervus cervicalis III
- H: rezeptives Feld des Nervus vagus

## Patentansprüche

1. Stimulationsvorrichtung (1) zur Stimulation mindestens eines rezeptiven Feldes eines Hautnervs des Kopf- oder Halsbereichs eines Menschen, wobei die Stimulationsvorrichtung (1) mindestens ein Stimulationselement (2) umfasst, das mit am Kopf (K) des Menschen anbringbaren Haltemitteln (3) in einer Lage gehalten werden kann, so dass eine Stimulationsoberfläche (4) des Stimulationselements (2) auf mindestens einem rezeptiven Feld aufliegt.

2. Stimulationsvorrichtung nach Anspruch 1, **dadurch gekennzeichnet, dass** die Stimulationsoberfläche (4) des Stimulationselements (2) flächig, insbesondere mit einer Auflagefläche von mindestens 2 cm², auf einem definierten Hautbereich aufliegt.

3. Stimulationsvorrichtung nach Anspruch 1 oder 2, **dadurch gekennzeichnet, dass** das Stimulationselement (2) mindestens eine elektrische Elektrode (5, 6) zur Aufbringung eines transkutanen elektrischen Stimulationsreizes auf den definierten Hautbereich aufweist.

4. Stimulationsvorrichtung nach Anspruch 1 oder 2, **dadurch gekennzeichnet, dass** das Stimulationselement (2) mindestens ein wärmeerzeugendes und/oder kälteerzeugendes Element zur Einleitung von Wärme und/oder Kälte auf den definierten Hautbereich aufweist.

5. Stimulationsvorrichtung nach Anspruch 1 oder 2, **dadurch gekennzeichnet, dass** das Stimulationselement (2) mindestens ein Schwingungen erzeugendes Element zur Einleitung von Vibrationen auf den definierten Hautbereich aufweist.

6. Stimulationsvorrichtung nach einem der Ansprüche 1 bis 5, **dadurch gekennzeichnet, dass** die Stimulationsoberfläche (4) des Stimulationselementes (2) zumindest auf einem Teil des Hautareals (9") über dem Processus mastoideus aufliegt.

7. Stimulationsvorrichtung nach einem der Ansprüche 1 bis 6, **dadurch gekennzeichnet, dass** die Haltemittel (3) einen am Stimulationselement (4) angeordneten oder angeformten bügelförmigen Abschnitt (7) umfassen, der ausgebildet ist, um hinter der Pinna (P) des Ohrs eingehängt zu werden.

8. Stimulationsvorrichtung nach einem der Ansprüche 1 bis 7, **dadurch gekennzeichnet, dass** die Haltemittel (3) einen am Stimulationselement (2) angeordneten oder angeformten Haltebügel (8) umfassen, der ausgebildet ist, um einen Teil des Kopfs (K), insbesondere um den Hinterkopf, umzulaufen und mindestens ein Stimulationselement (2), vorzugsweise zwei Stimulationselemente (2) für zwei rezeptive Felder, in Position zu halten.

9. Stimulationsvorrichtung nach Anspruch 8, **dadurch gekennzeichnet, dass** der Haltebügel (8) ausgebildet ist, um bei bestimmungsgemäßem Gebrauch der Stimulationsvorrichtung (1) das mindestens eine Stimulationselement (2), vorzugsweise zwei Stimulationselemente (2), mit Vorspannung an die Oberfläche des Kopfs (K) zu drücken.

10. Stimulationsvorrichtung nach einem der Ansprüche 1 bis 7, **dadurch gekennzeichnet, dass** die Haltemittel (3) einen schalenförmigen Körper (10) umfassen, der eine konkave Oberfläche aufweist, um einen Abschnitt des Kopfes (K) zu umfassen.

11. Stimulationselement nach Anspruch 10, **dadurch gekennzeichnet, dass** der schalenförmige Körper (10) aus einem Material besteht, das plastisch verformbar ist.

12. Stimulationsvorrichtung nach einem der Ansprüche 1 bis 7, **dadurch gekennzeichnet, dass** die Haltemittel (3) einen bandförmigen Streifen umfassen, an dem mindestens ein Stimulationselement (2) angeordnet ist, wobei der bandförmige Streifen insbesondere ausgebildet ist, über einen Teil des Kopfs (K) flächig zu verlaufen, wobei der bandförmige Streifen insbesondere aus einem Material besteht, das plastisch verformbar ist.

13. Stimulationselement nach Anspruch 12, **dadurch gekennzeichnet, dass** der bandförmige Streifen mindestens eine Gabelung aufweist, so dass er mindestens drei Abschnitte hat, wobei insbesondere jeder Abschnitt bei bestimmungsgemäßem Gebrauch des Stimulationselements über einen Umfangsabschnitt des Kopfs (K) verläuft.

14. Stimulationselement nach einem der Ansprüche 1 bis 13, **dadurch gekennzeichnet, dass** die Haltemittel (3) ausgebildet sind, um mindestens ein Stimulationselement (2) in mindestens einem der folgenden Bereiche des Kopfs (K) zu halten:
- Bereich des rezeptiven Feldes des Nervus ophthalmicus (A) (erster Ast des Nervus trigeminus) im Bereich der Stirn und des Scheitels,
- Bereich des rezeptiven Feldes des Nervus maxilliaris (B) (zweiter Ast des Nervus trigeminus) im Bereich der Wange und oberen Schläfe,
- Bereich des rezeptiven Feldes des Nervus mandibularis (C) (dritter Ast des Nervus trigeminus) am Kinn, Unterkiefer und unterer Schläfe bis oberhalb des Ohres,
- Bereich des rezeptiven Feldes des Nervus auricularis magnus (D) vom seitlichen Unterkiefer bis hinter das Ohr,
- Bereich des rezeptiven Feldes des Nervus occipitalis minor (E) hinter dem Ohr bis zur Hinterhauptschuppe,
- Bereich des rezeptiven Feldes des Nervus occipitalis major (F) an der Hinterhauptschuppe,
- Bereich des rezeptiven Feldes der Hautäste des Nervus cervicalis III (G) im Halsbereich von unterhalb der Kinnspitze bis zu den Sehnenansatzpunkten an der Hinterhauptschuppe,
- Bereich des rezeptiven Feldes des Nervus vagus (H, 9"), insbesondere des aurikulären Vagusnervs, im Bereich der Pinna (P) und über dem Mastoid.

15. Stimulationselement nach Anspruch 14, **dadurch gekennzeichnet, dass** die Haltemittel (3) ausgebildet sind, mindestens ein Stimulationselement (2) jeweils auf dem linken und rechten rezeptiven Feld zu halten.
